# FASCICULE DE BREVET EUROPEEN

(11) **EP 0 720 743 B1**
(45) Date de publication et mention de la délivrance du brevet: **02.01.1997**
(21) Numéro de dépôt: 94927705.7
(22) Date de dépôt: 20.09.1994
(51) Int. Cl.: G01N 33/50, G01N 27/72

(54) **PROCEDE ET DISPOSITIF POUR LA DETERMINATION D'UN ANALYTE DANS UN ECHANTILLON**
VERFAHREN UND VORRICHTUNG ZUR BESTIMMUNG EINES ANALYTEN IN EINER PROBE
METHOD AND DEVICE FOR DETERMINING AN ANALYTE IN A SAMPLE

(30) Priorité: 20.09.1993 FR 9311363
(43) Date de publication de la demande: 10.07.1996
(73) Titulaire: BIO MERIEUX, F-69280 Marcy l'Etoile (FR)
(72) Inventeur: COLIN, Bruno, F-69160 Tassin-la-Demi-Lune (FR); GOUDARD, Michel, F-69290 Saint-Genis-les-Ollières (FR); THERETZ, Alain, F-69130 Ecully (FR)
(74) Mandataire: Guerre, Dominique
(86) Numéro de dépôt international: FR9401099
(87) Numéro de publication internationale: WO9508769

(56) Documents cités:
- EP-A- 0 339 623
- GB-A- 2 074 727
- US-A- 4 913 883
- DATABASE WPI Week 8519, Derwent Publications Ltd., London, GB; AN 85-113585 & JP,A,60 055 265 (FUJI REBIO KK) 30 Mars 1985

## Description

La présente invention a pour objet un procédé pour la détermination d'un analyte susceptible d'être présent dans un échantillon.

Selon l'invention, par détermination d'un analyte, on entend la détection et/ou le dosage de toute substance notamment chimique, biochimique ou biologique.

L'analyte à déterminer peut être sans limitation un antigène, un haptène, un anticorps, un peptide, un fragment d'acide nucléique (ADN ou ARN), un enzyme, un substrat, à la condition que l'analyte comprenne un ligand ayant au moins un site de reconnaissance susceptible de se lier spécifiquement à un anti-ligand déterminé.

Selon la demande de brevet EP-0 339 623, il est décrit un procédé pour la détermination d'un analyte dans un échantillon, selon lequel :
i) on dispose dudit échantillon en phase liquide, dont l'analyte comprend un ligand ayant au moins un site de reconnaissance spécifique d'un anti-ligand
ii) on dispose d'au moins un réactif comprenant des particules métalliques ayant une taille de quelques nanomètres, en suspension en phase liquide, non séparables de ladite phase liquide par voie magnétique ou électromagnétique, chaque particule comprenant un noyau métallique sur lequel est fixé directement ou indirectement au moins un anti-ligand
iii) on met en contact, notamment par incubation, l'échantillon et le réactif, éventuellement avec un autre réactif, pour obtenir des agrégats métalliques
iv) on place les agrégats métalliques sous un champ magnétique, pour rassembler les agrégats métalliques
v) on détecte, par diffraction de lumière, les agrégats métalliques rassemblés, pour obtenir un signal représentatif de la masse métallique desdits agrégats, corrélé avec la quantité d'analyte présente à l'origine dans l'échantillon.

Le procédé conforme au document EP-A-0 339 623 présente l'inconvénient de recourir à un moyen de détection des agrégats métalliques rassemblés par le champ magnétique, particulièrement complexe, puisqu'en pratique il nécessite une source de lumière monochromatique cohérente (laser), un détecteur de la lumière diffractée, et un traitement du signal lumineux ainsi recueilli.

La présente invention a pour objet un procédé recourant à un dispositif de détection beaucoup plus simple, et permettant de corréler avec précision la masse métallique des agrégats métalliques rassemblés, avec la quantité d'analyte présente dans l'échantillon.

En effet, selon l'invention, on détecte la masse métallique desdits agrégats, à l'aide d'un capteur magnétique ou magnétisable, générant le champ ayant servi à rassembler les agrégats métalliques, ledit capteur étant placé au voisinage de l'échantillon, et soumis à un déplacement ou à une force sous l'effet des agrégats métalliques rassemblés, pour obtenir un signal de déplacement ou de force, représentatif de la masse métallique des agrégats.

Bien entendu, par capteur magnétique, on entend aussi tout capteur électromagnétique ou analogue.

Les termes ligand et anti-ligand tels qu'utilisés dans la présente invention font référence à toutes molécules biologiques susceptibles de former un complexe ligand/anti-ligand tels les complexes antigène/anticorps, anticorps/haptène, hormone/récepteur, protéine/anticorps, biotine/streptavidine, lectine/sucre, chélatant/molécule chélatée, hybride oligonucléotide/oligonucléotide, hybride olinucléotide/acide nucléique, enzyme/substrat ; étant entendu que lorsque le ligand est un fragment d'acide nucléique, il peut s'agir indifféremment d'un fragment d'ARN ou d'un ADN.

Par anticorps selon l'invention, on entend des anticorps monoclonaux, des anticorps polyclonaux, des fragments d'anticorps et des anticorps obtenus par recombinaison génétique.

Par particules non séparables en phase liquide par voie magnétique ou électromagnétique, on entend des particules qui, en l'absence de champ magnétique ou électromagnétique, ne sont pas susceptibles de sédimenter, et qui, n'étant pas sédimentées, en présence d'un champ magnétique ou électromagnétique, sont choisies sur le critère de la taille, pour ne pas être attirées par ce champ.

La mesure de la masse métallique des agrégats métalliques peut être faite pendant ou après la mise en contact de l'échantillon et du réactif.

Selon l'invention, le capteur magnétique peut être par exemple un aimant, solidaire du plateau d'une balance, et on mesure la masse métallique des agrégats métalliques, par pesée négative, c'est-à-dire par mesure de l'allégement dudit plateau.

Selon le procédé de l'invention, on dispose :
- d'un réactif primaire comprenant des particules métalliques en suspension en phase liquide, non séparables de ladite phase liquide par voie magnétique, chaque particule comprenant un noyau métallique sur lequel est fixé directement ou indirectement au moins un anti-ligand susceptible de réagir avec un site de reconnaissance du ligand de l'analyte
- d'un réactif secondaire comprenant des particules métalliques en suspension en phase liquide, non séparables de ladite phase liquide par voie magnétique, chaque particule comprenant un noyau métallique sur lequel est fixé directement ou indirectement au moins un autre anti-ligand susceptible de réagir avec un autre site de reconnaissance du ligand de l'analyte.

Dans un mode de mise en oeuvre du procédé de l'invention, le ligand de l'analyte comprend au moins deux sites de reconnaissance identiques ou différents de l'anti-ligand du réactif, suffisamment éloignés l'un de l'autre pour permettre de combiner un même ligand avec deux anti-ligands.

Dans un autre mode de mise en oeuvre, l'anti-ligand du réactif comprend deux sites récepteurs du ligand de l'analyte, suffisamment éloignés l'un de l'autre pour permettre de combiner un même anti-ligand avec deux ligands.

Selon une autre mise en oeuvre du procédé, l'anti-ligand du réactif comprend au moins deux sites récepteurs, et le ligand de l'analyte comprend au moins deux sites de reconnaissance, respectivement spécifiques des deux sites récepteurs du réactif.

Selon un mode particulier, le réactif primaire et le réactif secondaire sont identiques et le ligand de l'analyte comprend au moins deux sites de reconnaissance identiques.

Quand les réactifs primaire et secondaire sont identiques, le réactif primaire peut comprendre un deuxième anti-ligand différent du premier et susceptible de se lier à un site de reconnaissance du ligand de l'analyte différent du site reconnu par le premier anti-ligand.

Pour une détermination de l'analyte par compétition, on dispose d'un autre réactif dit de compétition, comprenant des particules en suspension en phase liquide, non séparables de ladite phase liquide par voie magnétique, chaque particule comprenant un noyau métallique sur lequel est fixé directement ou indirectement un autre ligand spécifiquement reconnu par l'anti-ligand du réactif.

Un autre objet de l'invention est un dispositif pour la détermination d'un analyte dans un échantillon, comprenant :
i) un contenant pour recevoir un échantillon à déterminer en phase liquide, dont l'analyte comprend un ligand ayant au moins un site de reconnaissance spécifique d'un anti-ligand
ii) avec au moins un réactif comprenant des particules métalliques en suspension en phase liquide, non séparables de ladite phase liquide par voie magnétique, chaque particule comprenant un noyau métallique sur lequel est fixé directement ou indirectement au moins un anti-ligand, ayant au moins un site récepteur d'un ligand
iii) l'échantillon, ledit réactif et éventuellement un autre réactif, étant mis en contact dans ledit contenant, notamment par incubation, pour obtenir des agrégats métalliques
iv) un moyen magnétique délivrant un champ magnétique, dans lequel sont placés les agrégats métalliques, pour rassembler ces derniers
v) un moyen de détection des agrégats métalliques rassemblés, pour obtenir un signal représentatif de la masse métallique des agrégats, corrélé avec la quantité d'analyte présente à l'origine dans l'échantillon,
ledit moyen de détection de la masse métallique comprenant un capteur magnétique ou magnétisable, qui comporte le moyen magnétique selon iv), est placé au voisinage de l'échantillon, et est soumis à un déplacement ou à une force sous l'effet des agrégats métalliques rassemblés, pour obtenir un signal de déplacement ou de force, représentatif de la masse métallique des agrégats.

Dans une réalisation particulière, le capteur est solidaire d'un plateau d'une balance, laquelle délivre un signal de pesée négative, correspondant à l'allégement dudit plateau, et représentatif de la masse métallique des agrégats.

A titre d'exemple, le noyau métallique des particules du réactif est choisi parmi les matériaux qui sont intrinsèquement magnétiques ou magnétisables, tels que les sels complexes et les oxydes, les borures, les sulfures de fer, de cobalt, de nickel et les éléments de terres rares, ayant une susceptibilité magnétique élevée tels que hématite et ferrites. Le noyau métallique des particules comprend des métaux purs ou des alliages comprenant un ou plusieurs de ces éléments. Le noyau métallique est choisi de préférence pour être dépourvu de magnétisme résiduel, et sa taille moyenne est comprise entre 5 et 30 nm, en particulier 10 et 20 nm. Le noyau métallique peut représenter de 5 à 100% en poids, en particulier de 25 à 65 % en poids, de la particule insoluble.

Les particules peuvent comprendre une enveloppe en plus du noyau métallique. La composition de l'enveloppe n'est pas critique, dans la mesure où elle rend possible la fixation de ligands et d'anti-ligands, et où elle est susceptible de présenter des interactions avec le noyau métallique. A titre d'exemple l'enveloppe peut être un polymère naturel modifié ou non chimiquement, par exemple un polysaccharide tel que l'agarose, le dextrane et des dérivés de cellulose tels que la carboxyméthylcellulose; une protéine telle que la gélatine et un polymère d'albumine; un polymère de synthèse modifié ou non chimiquement tels que les acides acryliques ou méthacryliques.

La taille moyenne de la particule du réactif est comprise entre 20 et 100 nm, en particulier entre 50 et 70 nm.

Le ou les réactifs sont mélangés avec un échantillon liquide qui est supposé contenir l'analyte et donc le ligand, et soumis par exemple à une incubation. La mise en contact de l'échantillon et du réactif est effectuée à un pH déterminé qui est fonction de la nature du ligand à détecter. Bien que la mise en contact puisse être exécutée dans une gamme de températures très large, de 2 à 95°C, la température avantageusement choisie est la température ambiante ou une température comprise entre 37°C et 40°C. Le temps de mise en contact est déterminé en fonction du type de dosage selon des conditions opératoires conventionnelles connues en soi.

Habituellement, un tampon est utilisé pour maintenir le pH désiré. La solution tampon pouvant être utilisée peut être choisie parmi un tampon d'acide phosphorique ou un tampon Tris etc... Dans certains cas, des sels, des protéines ou des détergents sont ajoutés au mélange pour éviter des réactions non spécifiques.

La présente invention est à présent illustrée par le schéma en annexe et les exemples suivants.

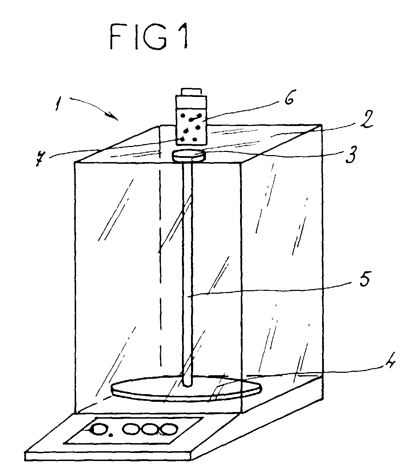

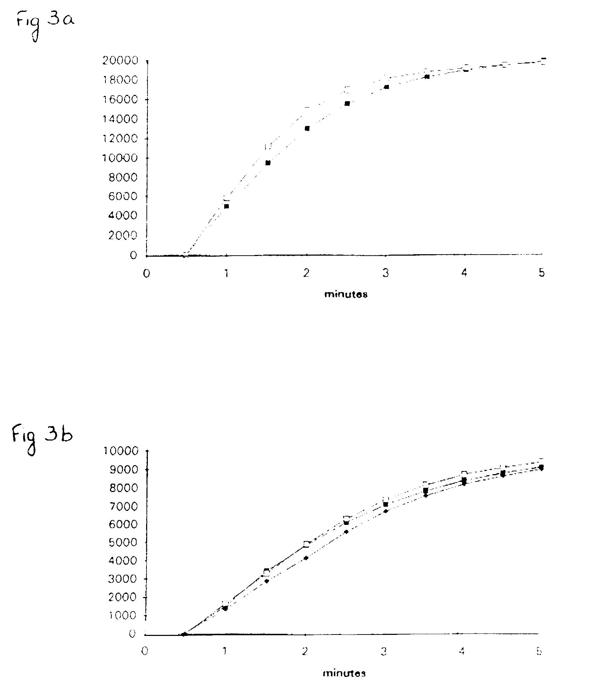

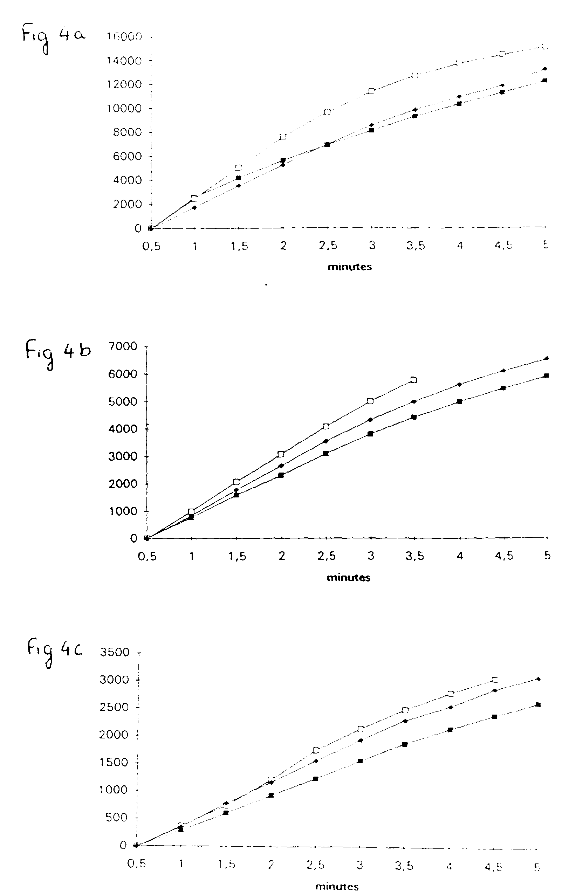

Le schéma comprend les figures 1 à 4 selon lesquelles:
Figure 1 représente un dispositif de détermination d'un analyte dans un échantillon selon l'invention,

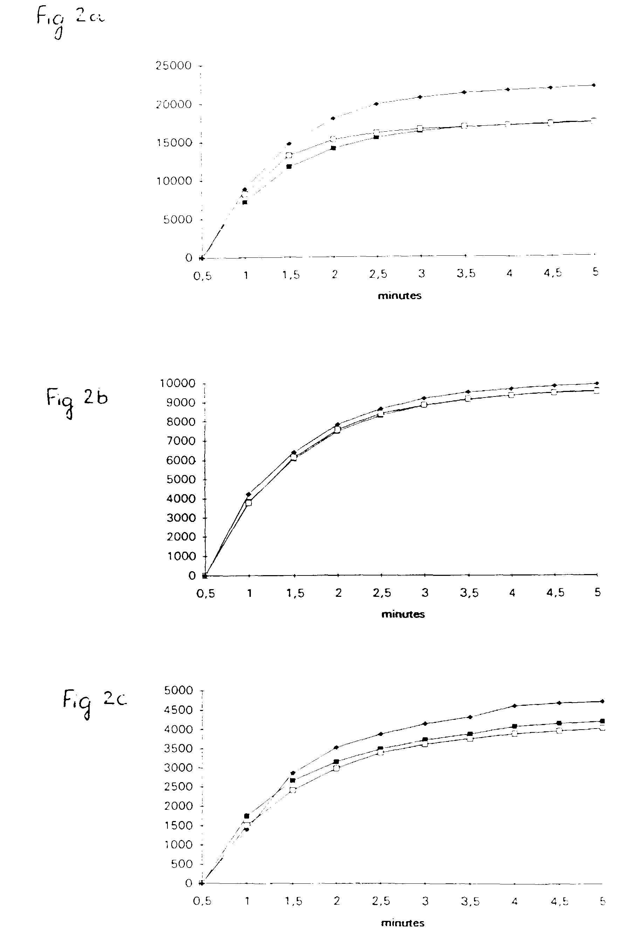

Figures 2, 3 et 4 correspondent aux courbes représentant des pesées négatives exprimées en unité magnétique arbitraire (en µg lus sur la balance), en fonction du temps, pour des concentrations d'anticorps anti-ferritine, de respectivement 100 µg, 25µg et 10µg par mg de particules métalliques. Pour chaque concentration, trois dilutions, 1/5 (a), 1/10 (b) et 1/20 (c) ont été testées, et pour chaque dilution, trois essais sont réalisés: un blanc (représenté sur les courbes par ■ ), un essai avec 10 µl de ferritine (représenté sur les courbes par □ ), et un essai avec 50 µl de ferritine (représenté sur les courbes par ◆ ).

Conformément à la Figure 1, un dispositif (1) de l'invention consiste en une balance de précision standard du commerce de type Mettler MT5, sous la vitre supérieure (2) de laquelle est placé un aimant (3) d'un diamètre de 13 mm et de 5 mm de hauteur, l'aimant étant solidaire du plateau (4) de la balance par l'intermédiaire d'un axe (5) vertical non magnétique. Un contenant (6) recevant l'échantillon à déterminer est placé sur une feuille d'aluminium placée au-dessus de l'aimant mais sans contact avec celui-ci. La distance entre l'aimant et le récipient est de 1 mm. L'échantillon et le réactif (7) sont mis en contact, dans le contenant (6).

### EXEMPLE 1: Détermination de ferritine

### a) Synthèse de particules Fe₃O₄/Dextran T40 (50 %)

14 grammes de Dextran T40 (Mw = 40.000, Pharmacia) sont ajoutés à 14 ml d'eau, et on laisse se dissoudre le Dextran à température ambiante, pour former une première solution.

Une deuxième solution est préparée avec 3 grammes FeCl₃, 6H₂O (Mw = 270,30) et 1,3 grammes FeCl₂, 4H₂O (Mw = 198,81), dans 20 ml d'eau.

Les deux solutions sont introduites dans un réacteur double enveloppe de 250 ml, équipé d'un moteur d'agitation réglé à environ 200-250 tpm, d'un agitateur en verre et d'une ampoule de coulée contenant une solution de NH4OH 7,5 % (v/v).

A température ambiante , on ajoute goutte à goutte la solution de NH₄OH 7,5 % (v/v) jusqu'à un pH final compris entre 10 et 11. Le réacteur est amené à 70°C pendant environ 60 minutes.A la fin de la réaction, la solution est récupérée et dialysée extensivement contre 5 1 d'eau distillée, puis filtrée sur laine de quartz. La solution est ensuite centrifugée 3 fois à 600 tpm pendant 5 minutes.

Afin d'éliminer le Dextran qui n'a pas réagi, les particules sont déposées sur une colonne de 33 x 2,5 cm de gel de Sephacryl S300 HR (Pharmacia), préalablement équilibrée par un tampon acétate 0.1 M, NaCl 0.15 M, 0,05% NaN₃, pH 6,5.

Les particules métalliques obtenues ont un diamètre extérieur compris entre 20 et 900 nm, et préférentiellement compris entre 50 et 70 nm, avec une enveloppe de Dextran. Elles sont conservées à +4°C.

### b) Préparation de particules portant un anticorps anti-ferritine.

Les particules sont soumises à trois dialyses contre un tampon PBS (0,15M, 2 litres, pH 6,8) respectivement pendant 2, 4 heures et une nuit. Ces particules sont oxydées à l'abri de la lumière, pendant environ 2 heures, par addition de périodate de sodium (0,1M) pour une concentration finale dans le milieu réactionnel de 10mM. Les particules sont ensuite soumises à deux nouvelles dialyses contre une solution de NaCl (0,15M, 2 litres) respectivement pendant environ 4 heures et une nuit. Le pH est ajusté par addition de NaHCO₃ à environ 8.

A la solution contenant les particules métalliques, sont ajoutés 100µg d'anticorps anti-ferritine (Bambou 8 fabriqué par la société CLONATEC). Le mélange est incubé de 5 heures à une nuit, à 25°C sous agitation douce. Le mélange ainsi formé est réduit par addition d'une solution de borohydrure de sodium (pour une concentration finale de 20mM dans le milieu réactionnel total), sous agitation douce pendant environ 30 minutes. Le mélange est dialysé 3 fois contre 2 litres d'un tampon phosphate de sodium (0,05 M) -NaCl 0,15 M, pH 7,5 pendant environ 2 heures. La solution finale contient 2,25 mg de particules métalliques par ml de solution, 10 à 100 µg d'anti-ferritine par mg de particule.

### c) Détermination de ferritine

Les solutions ont été testées comme décrit ci-après.

Les solutions qui comprennent 2,25 mg de particules par ml et respectivement 10, 25 et 100 µg d'anti-ferritine/mg de particules sont testées respectivement après dilution au 1/5ème, au 1/10ème et au 1/20ème, au moyen du dispositif décrit à la Figure 1.

Un prélèvement de 500 µl de chaque solution diluée est ajouté respectivement dans deux tubes en verre dans lesquels sont ajoutés respectivement 10 et 50µl de ferritine (2.000 mg/ml). Les solutions sont testées contre un blanc ne contenant pas de ferritine. La force d'attraction magnétique est mesurée au cours du temps. Les valeurs sont des valeurs de pesée négative données en unités magnétiques arbitraires.

Les résultats sont présentés dans les tableaux ci-après :

Concentration d'anticorps anti-ferritine: 100µg/mg de particules

| Dilution 1/5 | | | |
|---|---|---|---|
| Minutes | Blanc | 10µl | 50µl |
| 0,5 | 0 | 0 | 0 |
| 1 | 7200 | 8270 | 8900 |
| 1,5 | 11760 | 13270 | 14800 |
| 2 | 14160 | 15320 | 18050 |
| 2,5 | 15550 | 16160 | 19850 |
| 3 | 16350 | 16620 | 20710 |
| 3,5 | 16850 | 16910 | 21280 |
| 4 | 17120 | 17080 | 21620 |
| 4,5 | 17330 | 17200 | 21850 |
| 5 | 17440 | 17300 | 22000 |

| Dilution 1/10 | | | |
|---|---|---|---|
| Minutes | Blanc | 10µl | 50µl |
| 0,5 | 0 | 0 | 0 |
| 1 | 3830 | 3770 | 4230 |
| 1,5 | 6070 | 6160 | 6420 |
| 2 | 7480 | 7570 | 7840 |
| 2,5 | 8320 | 8430 | 8660 |
| 3 | 8850 | 8880 | 9220 |
| 3,5 | 9160 | 9200 | 9550 |
| 4 | 9390 | 9390 | 9730 |
| 4,5 | 9550 | 9520 | 9890 |
| 5 | 9650 | 9600 | 9990 |

| Dilution 1/20 | | | |
|---|---|---|---|
| Minutes | Blanc | 10µl | 50µl |
| 0,5 | 0 | 0 | 0 |
| 1 | 1750 | 1510 | 1400 |
| 1,5 | 2670 | 2420 | 2880 |
| 2 | 3170 | 3000 | 3540 |
| 2,5 | 3500 | 3400 | 3880 |
| 3 | 3740 | 3630 | 4160 |
| 3,5 | 3900 | 3780 | 4340 |
| 4 | 4110 | 3910 | 4640 |
| 4,5 | 4190 | 3990 | 4720 |
| 5 | 4250 | 4070 | 4760 |

Concentration d'anticorps anti-ferritine: 25µg/mg de particules

| Dilution 1/5 | | |
|---|---|---|
| Minutes | Blanc | 10µl |
| 0,5 | 0 | 0 |
| 1 | 4920 | 5800 |
| 1,5 | 9470 | 11200 |
| 2 | 13010 | 14830 |
| 2,5 | 15540 | 16950 |
| 3 | 17240 | 18160 |
| 3,5 | 18320 | 18830 |
| 4 | 19010 | 19250 |
| 4,5 | 19500 | 19530 |
| 5 | 19880 | 19730 |

| Dilution 1/10 | | | |
|---|---|---|---|
| Minutes | Blanc | 10µl | 50µl |
| 0,5 | 0 | 0 | 0 |
| 1 | 1540 | 1680 | 1360 |
| 1,5 | 3480 | 3340 | 2900 |
| 2 | 4900 | 4950 | 4200 |
| 2,5 | 6170 | 6380 | 5650 |
| 3 | 7160 | 7450 | 6800 |
| 3,5 | 7920 | 8240 | 7650 |
| 4 | 8480 | 8820 | 8280 |
| 4,5 | 8900 | 9200 | 8740 |
| 5 | 9210 | 9500 | 9110 |

| Dilution 1/20 | | | |
|---|---|---|---|
| Minutes | Blanc | 10µl | 50µl |
| 0,5 | 0 | 0 | 0 |
| 1 | 620 | 640 | 660 |
| 1,5 | 1230 | 1310 | 1300 |
| 2 | 1920 | 1960 | 1930 |
| 2,5 | 2470 | 2570 | 2510 |
| 3 | 2980 | 3080 | 3030 |
| 3,5 | 3410 | 3480 | 3450 |
| 4 | 3710 | 3780 | 3800 |
| 4,5 | 3960 | 4020 | 4050 |
| 5 | 4150 | 4190 | 4240 |

Concentration d'anticorps anti-ferritine: 10µg/mg de particules.

| Dilution 1/5 | | | |
|---|---|---|---|
| Minutes | Blanc | 10µl | 50µl |
| 0,5 | 0 | 0 | 0 |
| 1 | 2550 | 2420 | 1740 |
| 1,5 | 4150 | 5020 | 3500 |
| 2 | 5600 | 7570 | 5220 |
| 2,5 | 6900 | 9620 | 6950 |
| 3 | 8100 | 11340 | 8560 |
| 3,5 | 9300 | 12670 | 9850 |
| 4 | 10350 | 13700 | 10930 |
| 4,5 | 1125 | 14420 | 11830 |
| 5 | 12150 | 15040 | 13170 |

| Dilution 1/10 | | | |
|---|---|---|---|
| Minutes | Blanc | 10µl | 50µl |
| 0,5 | 0 | 0 | 0 |
| 1 | 770 | 1000 | 840 |
| 1,5 | 1590 | 2070 | 1780 |
| 2 | 2300 | 3070 | 2650 |
| 2,5 | 3090 | 4080 | 3540 |
| 3 | 3810 | 5020 | 4330 |
| 3,5 | 4430 | 5780 | 5000 |
| 4 | 5000 | | 5640 |
| 4,5 | 5490 | | 6120 |
| 5 | 5920 | | 6550 |

| Dilution 1/20 | | | |
|---|---|---|---|
| Minutes | Blanc | 10µl | 50µl |
| 0,5 | 0 | 0 | 0 |
| 1 | 287 | 364 | 350 |
| 1,5 | 595 | 734 | 776 |
| 2 | 920 | 1211 | 1163 |
| 2,5 | 1238 | 1758 | 1563 |
| 3 | 1563 | 2143 | 1938 |
| 3,5 | 1876 | 2499 | 2301 |
| 4 | 2155 | 2814 | 2568 |
| 4,5 | 2400 | 3074 | 2889 |
| 5 | 2628 | | 3103 |

### Exemple 2: Détermination de bactéries Listeria dans un échantillon biologique

**a) Les particules métalliques utilisées pour cet exemple, ont été préparées conformément au protocole décrit à l'Exemple 1.**
**b) Préparation des particules portant au moins un anticorps dirigé contre les *Listeria,* appelé ci-après Ac anti-L.**

Les particules synthétisées selon a) de l'Exemple 1 sont oxydées, par addition de périodate de sodium (0,1M).

Après agitation pendant 45 minutes, à l'abri de la lumière, les particules sont soumises à une dialyse, contre du NaCl 0,15M, pendant 4 heures.

A la solution contenant les particules métalliques, dont la concentration est de lmg de particules par mg de réactif, 10µg/ml d'Ac anti-L sont ajoutés; le pH est ajusté par addition de NaHCO₃ à environ 8.

Le mélange est incubé pendant 20 heures, sous agitation douce. Le mélange ainsi formé est réduit par addition d'une solution de borohydrure de sodium, selon une concentration de 12.10⁻³ moles NaBH₄ par mg de particules, sous agitation douce pendant 45 minutes. Le mélange est dialysé toute la nuit contre un tampon phosphate de sodium (0,1M)-NaCl (0,15M) à pH 7,5.

La solution finale contient 0,415mg de particules métalliques par ml de réactif, 15,4µg d'Ac anti-L par mg de particules, soit environ 6,4µg Ac anti-L par ml de réactif.

### c) Détermination des bactéries Listeria

Le réactif est testé sur un bouillon coeur/cervelle, identifié par la référence : L. INNO ATCC, 410A 4, Lot/Ch-b 390853.

La détermination est comme pour l'Exemple 1, effectuée sur une balance METTLER MT5 dans les conditions suivantes :
- Contenant :: tube en verre de diamètre 10 mm, et de hauteur 48 mm,
- Aimant :: de diamètre 13 mm et de hauteur 5 mm, nord dirigé vers le haut
- Distance aimant-tube :: 1 mm (environ)

On a testé une première solution appelée T2 comprenant 500µl du réactif décrit en b) et 10µl de bouillon, après agitation et incubation, puis une seconde solution appelée T3 comprenant 500µl du réactif décrit en b), et qui sera utilisé comme témoin.

Les résultats présentés dans le tableau suivant correspondent aux valeurs de la mesure de la force d'attraction magnétique lues aux temps To et To+4heures, le temps To étant le temps de début de réaction, c'est-à-dire de positionnement du contenant au-dessus de l'aimant.

| **Temps de réaction** | **T2** | **T3** |
|---|---|---|
| To | -6000 | -6000 |
| To+4h | -18000 | -6000 |

La force d'attraction magnétique des agrégats métalliques dans l'échantillon T2 est donc de -12000, et sera corrélée à la concentration en bactéries au moyen d'une courbe étalon.

## Revendications

1. Procédé pour la détermination d'un analyte dans un échantillon, selon lequel :
i) on dispose dudit échantillon en phase liquide, dont l'analyte comprend un ligand ayant au moins un site de reconnaissance spécifique d'un anti-ligand
ii) on dispose d'au moins un réactif comprenant des particules métalliques en suspension en phase liquide, non séparables de ladite phase liquide par voie magnétique ou électromagnétique, chaque particule comprenant un noyau métallique sur lequel est fixé directement ou indirectement au moins un anti-ligand
iii) on met en contact, notamment par incubation, l'échantillon et le réactif, éventuellement avec un autre réactif, pour obtenir des agrégats métalliques
iv) on place les agrégats métalliques sous un champ magnétique, pour rassembler les agrégats métalliques
v) on détecte les agrégats métalliques rassemblés, pour obtenir un signal représentatif de la masse métallique desdits agrégats, corrélé avec la quantité d'analyte présente à l'origine dans l'échantillon
**caractérisé en ce que** on détecte la masse métallique des agrégats métalliques, à l'aide d'un capteur magnétique ou magnétisable, générant le champ selon iv), placé au voisinage de l'échantillon, et soumis à un déplacement ou à une force sous l'effet des agrégats métalliques rassemblés, pour obtenir un signal de déplacement ou de force, représentatif de la masse métallique des agrégats.

2. Procédé selon la revendication 1, caractérisé en ce que le capteur magnétique est solidaire du plateau d'une balance, et on mesure la masse métallique par pesée négative, c'est-à-dire par mesure de l'allégement dudit plateau.

3. Procédé selon la revendication 1, caractérisé en ce qu'on dispose :
- d'un réactif primaire comprenant des particules métalliques en suspension en phase liquide, non séparables de ladite phase liquide par voie magnétique, chaque particule comprenant un noyau métallique sur lequel est fixé directement ou indirectement au moins un anti-ligand susceptible de réagir avec un site de reconnaissance du ligand de l'analyte
- d'un réactif secondaire comprenant des particules métalliques en suspension en phase liquide, non séparables de ladite phase liquidepar voie magnétique, chaque particule comprenant un noyau métallique sur lequel est fixé directement ou indirectement au moins un autre anti-ligand susceptible de réagir avec un autre site de reconnaissance du ligand de l'analyte.

4. Procédé selon la revendication 1, caractérisé en ce que le ligand de l'analyte comprend au moins deux sites de reconnaissance de l'anti-ligand du réactif, suffisamment éloignés l'un de l'autre pour permettre de combiner un même ligand avec deux anti-ligands.

5. Procédé selon la revendication 4, caractérisé en ce que le ligand de l'analyte comprend au moins deux sites de reconnaissance identiques de l'anti-ligand du réactif.

6. Procédé selon la revendication 1, caractérisé en ce que l'anti-ligand du réactif comprend deux sites récepteurs du ligand de l'analyte, suffisamment éloignés l'un de l'autre pour permettre de combiner un même anti-ligand avec deux ligands.

7. Procédé selon la revendication 1, caractérisé en ce que l'anti-ligand du réactif comprend au moins deux sites récepteurs, et le ligand de l'analyte comprend au moins deux sites de reconnaissance, respectivement spécifiques des deux sites récepteurs du réactif.

8. Procédé selon les revendications 3 et 5, caractérisé en ce que le réactif primaire et le réactif secondaire sont identiques.

9. Procédé selon la revendication 1, selon lequel on détermine l'analyte par compétition, caractérisé en ce qu'on dispose d'un autre réactif dit de compétition, comprenant des particules en suspension en phase liquide, non séparables de ladite phase liquide par voie magnétique, chaque particule comprenant un noyau métallique sur lequel est fixé directement ou indirectement un autre ligand spécifiquement reconnu par l'anti-ligand du réactif.

10. Dispositif pour la détermination d'un analyte dans un échantillon, comprenant :
i) un contenant pour recevoir un échantillon à déterminer en phase liquide, dont l'analyte comprend un ligand ayant au moins un site de reconnaissance spécifique d'un anti-ligand
ii) avec au moins un réactif comprenant des particules métalliques en suspension en phase liquide, non séparables de ladite phase liquide par voie magnétique, chaque particule comprenant un noyau métallique sur lequel est fixé directement ou indirectement au moins un anti-ligand, ayant au moins un site récepteur d'un ligand
iii) l'échantillon, ledit réactif et éventuellement un autre réactif, étant mis en contact dans ledit contenant, notamment par incubation, pour obtenir des agrégats métalliques
iv) un moyen magnétique délivrant un champ magnétique, dans lequel sont placés les agrégats métalliques, pour rassembler ces derniers
v) un moyen de détection des agrégats métalliques rassemblés, pour obtenir un signal représentatif de la masse métallique des agrégats, corrélé avec la quantité d'analyte présente à l'origine dans l'échantillon
**caractérisé en ce que** le moyen de détection de la masse métallique comprend un capteur magnétique ou magnétisable, comportant le moyen magnétique selon iv), placé au voisinage de l'échantillon, et soumis à un déplacement ou à une force sous l'effet des agrégats métalliques rassemblés, pour obtenir un signal de déplacement ou de force, représentatif de la masse métallique des agrégats.

11. Dispositif selon la revendication 10, caractérisé en ce que le capteur est solidaire d'un plateau (4) d'une balance, laquelle délivre un signal de pesée négative, correspondant à l'allégement dudit plateau, et représentatif de la masse métallique des agrégats.

## Claims

1. A method for determining an analyte in a sample which comprises:
i) providing said sample in liquid phase, the analyte of which comprises a ligand having at least one anti-ligand specific recognition site;
ii) providing at least one reagent containing metal particles, in suspension in liquid phase and non-separable from said liquid phase by magnetic or electromagnetic means, each particle containing a metal core to which at least one anti-ligand is fixed directly or indirectly;
iii) contacting, especially by incubation, the sample and the reagent, optionally with another reagent, in order to obtain metal clusters;
iv) placing the metal clusters in a magnetic field, in order to assemble the metal clusters;
v) detecting the assembled metal clusters to obtain a signal representative of the mass of metal in said clusters, correlated with the quantity of analyte present in the sample initially
**wherein** the metal mass of the said clusters is detected using a magnetic or magnetizable sensor generating the field according to iv), the said sensor being placed close to the sample and moved or subjected to a force caused by the assembled metal clusters, giving a movement or force signal representative of the metal mass of the clusters.

2. The method as claimed in claim 1, wherein the magnetic sensor is integrally attached to the tray of a balance, and the metal mass is measured by negative weighing, that is to say by measuring the lightening of said tray.

3. The method as claimed in claim 1, wherein there is provided:
- a primary reagent comprising metal particles in suspension in liquid phase, non-separable from said liquid phase by magnetic means, each particle comprising a metal core to which is fixed directly or indirectly at least one anti-ligand capable of reacting with a recognition site of the analyte ligand
- a secondary reagent comprising metal particles in suspension in liquid phase and non-separable from said liquid phase by magnetic means, each particle containing a metal core to which is fixed directly or indirectly at least one other anti-ligand capable of reacting with another recognition site of the analyte ligand.

4. The method as claimed in claim 1, wherein the analyte ligand comprises at least two recognition sites of the reagent anti-ligand which are sufficiently far apart from each other to allow the same ligand to be combined with two-anti-ligands.

5. The method as claimed in claim 4, wherein the analyte ligand comprises at least two identical recognition sites of the reagent anti-ligand.

6. The method as claimed in claim 1, wherein the reagent anti-ligand comprises two receptor sites of the analyte ligand which are sufficiently far apart from each other to allow the same anti-ligand to be combined with two ligands.

7. The method as claimed in claim 1, wherein the reagent anti-ligand comprises at least two receptor sites, and the analyte ligand comprises at least two recognition sites which are specific for the two receptor sites of the reagent respectively.

8. The method as claimed in claims 3 and 5, wherein the primary reagent and the secondary reagent are identical.

9. The method as claimed in claim 1, according to which the analyte is determined by competition, wherein another so-called competition reagent is provided containing particles in suspension in liquid phase and non-separable from said liquid phase by magnetic means, each particle comprising a metal core to which is fixed directly or indirectly another ligand specifically recognized by the reagent anti-ligand.

10. A device for determining an analyte in a sample, comprising:
i) a container for receiving a sample to be determined in liquid phase, the analyte of which comprises a ligand having at least one anti-ligand specific recognition site
ii) with at least one reagent comprising metal particles in suspension in liquid phase and non-separable from said liquid phase by magnetic means, each particle containing a metal core to which is fixed directly or indirectly at least one anti-ligand, having at least one receptor site of a ligand
iii) the sample, said reagent and optionally another reagent being contacted in said container, especially by incubation, in order to obtain metal clusters
iv) a magnetic means delivering a magnetic field, in which the metal clusters are placed in order to assemble them
v) a means for detecting the assembled metal clusters, in order to obtain a signal representative of the mass of metal in the clusters, correlated with the quantity of analyte present in the sample initially,
wherein said means for detecting the metal mass comprises a magnetic or magnetizable sensor, which comprises the magnetic means according to iv), placed close to the sample, and moved or subjected to a force caused by the assembled metal clusters, giving a movement or force signal representative of the metal mass of the clusters.

11. The device as claimed in claim 10, wherein the sensor is integrally attached to a tray of a balance which delivers a negative weighing signal corresponding to the lightening of said tray, and representative of the metal mass of the clusters.

## Patentansprüche

1. Verfahren zur Bestimmung eines Analyten in einer Probe, in dem:
i) die Probe in eine flüssige Phase gebracht wird, wobei der Analyt einen Liganden enthält, der zumindest eine für einen Anti-Liganden spezifische Erkennungsstelle aufweist
ii) zumindest ein reaktives Agens bereitgestellt wird, das metallische Partikel in Suspension in flüssiger Phase enthält, die von der flüssigen Phase nicht magnetisch oder elektromagnetisch trennbar sind, wobei jedes Partikel einen Metallkern aufweist, auf dem direkt oder indirekt zumindest ein Anti-Ligand fixiert ist
iii) die Probe und das reaktive Agens insbesondere durch Inkubation, und gegebenenfalls mit einem weiteren reaktiven Agens in Kontakt gebracht werden, um metallische Aggregate zu erhalten
iv) die metallischen Aggregate in ein Magnetfeld gebracht werden, um die metallischen Aggregate zu sammeln
v) die gesammelten metallischen Aggregate detektiert werden, um ein Signal zu erhalten, das der Masse an Metall der Aggregate entspricht und das mit der ursprünglich in der Probe enthaltenen Menge des Analyten korreliert
**dadurch gekennzeichnet**, daß die Masse an Metall der metallischen Aggregate mit Hilfe eines magnetischen oder magnetisierbaren Meßfühlers detektiert wird, der das Feld gemäß iv) erzeugt, und der in der Umgebung der Probe plaziert ist, und der einer Verlagerung oder einer Kraft unter der Wirkung der gesammelten metallischen Aggregate ausgesetzt ist, um ein Verlagerungs- oder Kraftsignal zu erhalten, das der Masse an Metall der Aggregate entspricht.

2. Verfahren nach Anspruch 1, dadurch gekennzeichnet, daß der magnetische Meßfühler fest mit der Waagschale einer Waage verbunden ist, und daß die Masse an Metall durch negatives Wiegen, d.h. durch Messung der Gewichtsverringerung der Waagschale, gemessen wird.

3. Verfahren nach Anspruch 1, dadurch gekennzeichnet, daß
- ein primäres reaktives Agens bereitgestellt wird, das metallische Partikel in Suspension in flüssiger Phase enthält, die nicht magnetisch von der flüssigen Phase trennbar sind, wobei jedes Partikel einen Metallkern aufweist, auf dem direkt oder indirekt zumindest ein Anti-Ligand fixiert ist, der zur Reaktion mit einer Erkennungsstelle des Liganden des Analyten geeignet ist, und daß
- ein sekundäres reaktives Agens bereitgestellt wird, das metallische Partikel in Suspension in flüssiger Phase enthält, die nicht magnetisch von der flüssigen Phase trennbar sind, wobei jedes Partikel einen Metallkern aufweist, auf dem direkt oder indirekt zumindest ein weiterer Anti-Ligand fixiert ist, der zur Reaktion mit einer weiteren Erkennungsstelle des Liganden des Analyten geeignet ist.

4. Verfahren nach Anspruch 1, dadurch gekennzeichnet, daß der Ligand des Analyten zumindest zwei Erkennungsstellen für den Anti-Liganden des reaktiven Agens aufweist, die genügend weit voneinander entfernt sind, um ein und denselben Liganden mit zwei Anti-Liganden zu verbinden.

5. Verfahren nach Anspruch 4, dadurch gekennzeichnet, daß der Ligand des Analyten zumindest zwei identische Erkennungsstellen für den Anti-Liganden des reaktiven Agens aufweist.

6. Verfahren nach Anspruch 1, dadurch gekennzeichnet, daß der Anti-Ligand des reaktiven Agens zumindest zwei Rezeptorstellen für den Liganden des Analyten aufweist, die genügend weit voneinander entfernt sind, um ein und denselben Anti-Liganden mit zwei Liganden zu verbinden.

7. Verfahren nach Anspruch 1, dadurch gekennzeichnet, daß der Anti-Ligand des reaktiven Agens zumindest zwei Rezeptorstellen aufweist, und daß der Ligand des Analyten zumindest zwei Erkennungsstellen aufweist, die jeweils spezifisch für die zwei Rezeptorstellen des reaktiven Agens sind.

8. Verfahren nach den Ansprüchen 3 und 5, dadurch gekennzeichnet, daß das primäre reaktive Agens und das sekundäre reaktive Agens identisch sind.

9. Verfahren nach Anspruch 1, bei dem der Analyt durch Kompetition bestimmt wird, dadurch gekennzeichnet, daß ein weiteres reaktives Agens, ein sogenannter Kompetitor vorgesehen ist, der Partikel in Suspension in flüssiger Phase enthält, die von der flüssigen Phase nicht magnetisch trennbar sind, wobei jeder Partikel einen Metallkern aufweist, auf dem direkt oder indirekt ein weiterer Ligand fixiert ist, der spezifisch von dem Anti-Liganden des reaktiven Agens erkannt wird.

10. Vorrichtung zur Bestimmung eines Analyten in einer Probe, enthaltend:
i) einen Behälter zur Aufnahme einer zu bestimmenden Probe in flüssiger Phase, wobei der Analyt einen Liganden enthält, der zumindest eine für einen Anti-Liganden spezifische Erkennungsstelle aufweist,
ii) zumindest ein reaktives Agens, das metallische Partikel in Suspension in flüssiger Phase enthält, die von der flüssigen Phase nicht magnetisch trennbar sind, wobei jedes Partikel einen Metallkern aufweist, auf dem direkt oder indirekt zumindest ein Anti-Ligand fixiert ist, der zumindest eine Rezeptorstelle für den Liganden aufweist
iii) wobei die Probe, das reaktive Agens und ggf. ein weiteres reaktives Agens in dem Behälter insbesondere durch Inkubation in Kontakt gebracht werden, um metallische Aggregate zu erhalten
iv) ein magnetisches Mittel, das ein magnetisches Feld erzeugt, in das die metallischen Aggregate eingebracht werden, um letztere zu sammeln
v) ein Mittel zur Detektion der gesammelten metallischen Aggregate, um ein Signal zu erhalten, das der Masse an Metall der Aggregate entspricht, und das mit der ursprünglich in der Probe enthaltenenen Menge des Analyten korreliert,
dadurch gekennzeichnet, daß das Mittel zur Detektion der Masse an Metall einen magnetischen oder magnetisierbaren Meßfühler aufweist, der das magnetische Mittel gemäß iv) enthält, der ferner in der Umgebung der Probe plaziert ist, und der einer Verlagerung oder einer Kraft unter Einwirkung der gesammelten metallischen Aggregate ausgesetzt wird, um ein Verlagerungs- oder Kraftsignal zu erhalten, das der Masse an Metall der Aggregate entspricht.

11. Vorrichtung nach Anspruch 10, dadurch gekennzeichnet, daß der Meßfühler fest mit einer Waagschale (4) einer Waage verbunden ist, die ein negatives Wiegesignal erzeugt, das der Gewichtsverringerung der Waagschale entspricht und das die Masse an Metall der Aggregate darstellt.
